# EUROPEAN PATENT SPECIFICATION

(11) **EP 0 816 509 B1**
(45) Date of publication and mention of the grant of the patent: **17.09.2003**
(21) Application number: 97670002.1
(22) Date of filing: 14.03.1997
(51) Int. Cl.: C12P 19/44, C12N 9/96, C12N 1/04, C12N 9/04, C07K 1/14

(54) **Thermostabilization, osmoprotection, and protection against desiccation of enzymes, cell components and cells by mannosyl-glycerate**
Thermostabilisierung, Osmoseschütz und Trocknungsschütz von Enzymen, Zellinhaltstoffen und Zellen mit Mannosyl-Glycerat
Thermostabilisation, osmoprotection et protection contre la dessication d'enzymes, de la matière de cellules et de cellules avec le mannosylglycérate

(30) Priority: 28.06.1996 PT 10188796
(43) Date of publication of application: 07.01.1998
(73) Proprietor: bitop Aktiengesellschaft für biotechnische Optimierung, 58453 Witten (DE)
(72) Inventor: Santos, Maria Helena Dias, 2780 Oeiras (PT); Martins, Ana Alexandra, 2780 Oeiras (PT); Costa, Milton, 3000 Coimbra (PT)
(74) Representative: Thiel, Christian, Dr. Dipl.-Chem.

(56) References cited:
- MARTINS, LIGIA O. ET AL: "Accumulation of mannosylglycerate and Di-myo-inositol-phosphate by Pyrococcus furiosus in response to salinity and temperature." APPLIED AND ENVIRONMENTAL MICROBIOLOGY, vol. 61, no. 9, September 1995 (1995-09), pages 3299-3303, XP002114722
- NUNES, OLGA C. ET AL: "Compatible Solutes in the Thermophilic Bacteria Rhodothermus marinus and "Thermus thermophilus." APPLIED AND ENVIRONMENTAL MICROBIOLOGY, vol. 61, no. 6, June 1995 (1995-06), pages 2351-2357, XP002114721
- MARTINS L O ET AL.: "Organic solutes in hyperthermophilic archae" APPLIED AND ENVIRONMENTAL MICROBIOLOGY, vol. 63, no. 3, 4 March 1997 (1997-03-04), pages 896-902, XP002115174
- RAMOS, ANA ET AL: "Stabilization of enzymes against thermal stress and freeze-drying by mannosylglycerate." APPLIED AND ENVIRONMENTAL MICROBIOLOGY, vol. 63, no. 10, October 1997 (1997-10), pages 4020-4025, XP002114723

## Description

The invention concerns the use of mannosylglycerate (either the β or the α anomers) for the stabilization of enzymes, other cellular components, and cells against general stress, namely caused by heat, high osmolarity, desiccation, freeze-drying, and repetitive use.

Several intracellular, small molecular weight, solutes are known to accumulate in high levels when microorganisms are subjected to extreme environmental conditions such as temperature or osmotic stress. These solutes generally serve as osmolytes in halotolerant and halophilic organisms.

Osmolytes, commonly designated compatible solutes, maintain turgor pressure and cell volume, and permit normal enzyme activity under osmotic stress. The extremely halophilic *Archaea* and a few halophilic anaerobic *Bacteria,* accumulate K , Na and Cl in response to changes in the extracellular salt concentration, while all other microorganisms examined utilize organic compatible solutes for osmotic adjustment to water stress. The number of compatible solutes is relatively small, and fall into certain categories of compounds such as polyols and derivatives, sugars and derivatives, amino acids and amino acid derivatives, small peptides, betaines, and ectoines [Brown, A. D. 1990. Microbial Water Stress Physiology - Principles and Perspectives. John Wiley & Sons, Chichester].

In addition to being compatible with the normal activities of the cell, osmolytes also seem to have a protective effect on the function of cell components, namely proteins. It has been shown that some compatible solutes are capable of stabilizing proteins against temperature denaturation and denaturing agents such as detergents, organic solvents, urea and guanidine chloride [Arakawa, T. and S. N. Timasheff. 1985. Biophys. J. **47**: 411-414; Arakawa, T. and S. N. Timasheff. 1983. Arch. Biochem. Biophys. **224**: 169-177; Arakawa, T. and S. N. Timasheff. 1982. Biochem. J. **21**: 6536-6544].

The addition of small molecular weight solutes to solutions containing proteins leads to alterations in the physical properties of water by increasing the surface tension and excluding solutes from the water-protein interface. In this way, the structure of water in the hydration shell of the protein becomes highly organized leading to the formation of a smaller protein-water interface. The area of globular proteins is smaller in the native state than in the denatured state, leading to a favourable ratio of native to denatured states.

The greater stability of proteins in the presence of certain small molecular weight organic solutes allows enzymes to function under more extreme conditions imposed by temperature, pressure, ionic strength, pH, detergents and organic solvents. The ability of some compatible solutes to stabilize enzymes is, therefore, of the utmost importance to modern biotechnology. It must also be stressed that compatible solutes protect proteins [Carpenter, J. F., J. H. Crowe and T. Arakawa. 1990. J. Dairy Sci. **73**: 3627-3636], cell membranes [Rudolph, A. S., J. H. Crowe and L. M. Crowe. 1986. Arch. Biochem. Biophys. **245**: 134-143] and cells [Louis, P., H. G. Truper and E. A. Galinski. 1994. Appl. Microbiol. Biotechnol. **41**: 684-688; Leslie, S. B., S. A. Teter, L. M. Crowe and J. H. Crowe. 1994. Biochim. Biophys. Acta **1192**: 7-13] from the deleterious effects of desiccation, and in spite of the great importance of desiccation and freezing in the conservation of biological samples, denaturation of proteins or a decrease of the viable count of cultures inevitably takes place. The preservation of desiccated or lyophilized cell components has great applications in medicine (conservation of tissues and other products of biological origin), in the pharmaceutical industry (microencapsulation of medicines), the cosmetic industry (liposomes), in the food industry (conservation of food), and scientific research (maintenance of cultures and cell lines).

Dehydration of cell membranes and cells results in a phase transition of the lipid bilayer. After rehydration, the membranes undergo a reverse phase transition that can lead to the rupture of the membrane. The protection of membranes by low molecular weight solutes is related to the substitution of water by these solutes between the polar head groups, thereby maintaining the appropriate lipid phase when these structures are dehydrated.

Some thermophilic and hypertermophilic *Bacteria* and *Archaea* also produce low molecular weight solutes. These solutes generally accumulate during growth at supraoptimal temperatures and/or salinities leading to the suggestion that they exert a protective effect on, at least, some cell components. Under high growth temepratures, di-myo-inositol-(1,1')-phosphate and cyclic 2,3'- diphosphoglycerate accumulate to high levels in *Pyrococcus furiosus* and *Methanothermus fervidus,* respectively [Martins, L. O. and H. Santos. 1995. Appl. Environ. Microbiol. **61**: 3299-3303; Hensel, R. and H. Konig. 1988. FEMS Microbiol. Lett. **49**: 75-79].

A novel compatible solute designated 2-O-β-mannosylglycerate accumulates in the archaeon *Pyrococcus furiosus,* and the bacteria *Rhodothermus marinus* and *Thermus thermophilus* subjected to salt stress [Martins, L. O. and H. Santos. 1995. Appl. Environ. Microbiol. **61**: 3299-3303; Nunes, O. C., C. M. Manaia, M. S. da Costa and H. Santos. 1995. Appl. Environ. Microbiol. **61**: 2351-2357]. In the three species the intracellular concentration of this solute increases concomitantly with the salinity of the growth medium. 2-O-β-Mannosylglycerate was also detected in species of the genera *Methanothermus* and *Thermococcus,* [Martins, L. O., R. Huber, H. Huber, K. O. Stetter, M. S. da Costa and H. Santos. 1997. Appl. Environ. Microbiol. 63: 000-000].

It is expected that the α-anomer of mannosylglycerate exhibits properties similar to those of the β-anomer. The α-anomer of mannosylglycerate was identified previously in species of the algal order Ceramiales [Kremer, B. P. and R. Vogl, 1975. Phytochem. **14**: 1309-1314], and also in the thermophilic bacteria *Rhodothermus marinus,* [Nunes, O. C., C. M. Manaia, M. S. da Costa and H. Santos. 1995. Appl. Environ. Microbiol. **61**: 2351-2357].

2-*O*-β-Mannosylglycerate is considerably better than trehalose as a thermoprotectant for enzymes and is at least as good as trehalose in protecting enzymes against stress imposed by desiccation. This product can be used as a stabilizing agent of several biological systems, such as, proteins, enzymes, biological membranes or artificial membranes or cells, vulnerable to damage and decreased efficiency (or viability) under harsh or stressing conditions, such as high temperatures, high salt concentrations, desiccation and/or freezing, and the action of denaturing agents (organic solvents, detergents, oxidizing agents and chaotropic substances. In addition, mannosylglycerate can be used as an additive in cosmetics, either for stabilization of liposomes or for the improvement of moisturizing properties.

### BRIEF DESCRIPTION OF THE DRAWINGS

**Figure 1** depicts the chemical structure of 2-*O*-β-mannosylglycerate. It is based on the data of Nunes, O. C., C. M. Manaia, M. S. da Costa and H. Santos. 1995. Appl. Environ. Microbiol. **61**: 2351-2357.
**Figure 2** shows a comparison between the proton NMR spectrum of an extract (A) and that of purified 2-*O*-β-mannosylglycerate (B).
**Figure 3** shows the effect of 2-*O*-β-mannosylglycerate on the thermostability of rabbit muscle LDH (A), baker's yeast ADH (B), and bovine liver GDH (C).
**Figure 4** shows the effect of the concentration of 2-*O*-β-mannosylglycerate (●) and KC1 ( ) on the thermal stability of rabbit muscle LDH after 10 min incubation at 50°C.
**Figure 5** shows the effect of 2-*O*-β-mannosylglycerate on enzymic activity after freeze-drying rabbit muscle LDH (A) and yeast ADH (B).

### EXAMPLE 1

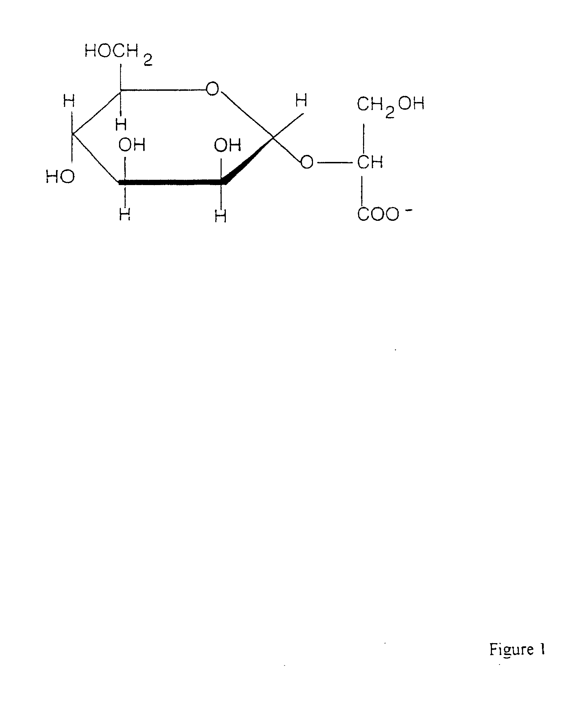

This example shows that 2-*O*-β-mannosylglycerate (Figure 1) can be purified from ethanolic extracts of *Pyrococcus furiosus, Pyrococcus woesei, Methanothermus* spp, *Thermococcus* spp, *Rhodothermus marinus,* and Thermus *thermophilus* after growth of these organisms in complex medium at supraoptimal temperature and salinities. 2-*O*-β-Mannosylglycerate was purified from ethanol/chloroform extracts of *Pyrococcus furiosus* by anion-exchange chromatography on QAE-Sephadex A25. The extract, corresponding to approximately 100 g cells (wet weight) was loaded onto a QAE-Sephadex column (5 × 20 cm) equilibrated with 5 mM ammonium bicarbonate, pH 8.0. The elution was performed with a linear gradient of 5 mM to 1 M NH₄HCO₃ at a flow rate of 1 ml. min⁻¹ . Fractions containing carbohydrates or phosphorus were pooled separately, concentrated by lyophylization and analyzed by ¹H-NMR spectroscopy. Three main carbohydrate containing fractions were eluted: the first fraction eluted at 5 mM NH₄HCO₃ and contained a non-identified polysaccharide; the second fraction eluted between 0.6-0.7 M NH₄HCO₃ and contained a mixture of di-myo-inositol-phosphate and *2-O-β*-mannosylglycerate, and the third fraction, eluted at 0.8 M NH₄HCO₃ contained pure 2-*O*-β-mannosylglycerate as judged by ¹H-NMR spectroscopy. The fractions containing a mixture of di-myo-inositol-phosphate and 2-*O*-β-mannosylglycerate were concentrated by lyophilization, loaded onto a QAE-Sephadex column (2.6×20 cm) and eluted as described above; the fractions containing pure 2-*O*-β-mannosylglycerate were pooled, and NH₄HCO₃ was partially removed by passage through the ion retardation resin AG 11-A8 (2 × 40 cm). Residual NH₄⁺ was exchanged by H⁺ by passage through a Dowex AG 50W-X8 column (2.5 x 10 cm). The K⁺ salt of 2-*O*-β-mannosylglycerate was obtained by neutralization with KOH, the sample was lyophilized and resuspended in ultra pure water in order to obtain a 1 M solution of 2-*O*-β-mannosylglycerate (potassium salt), pH 7.0.

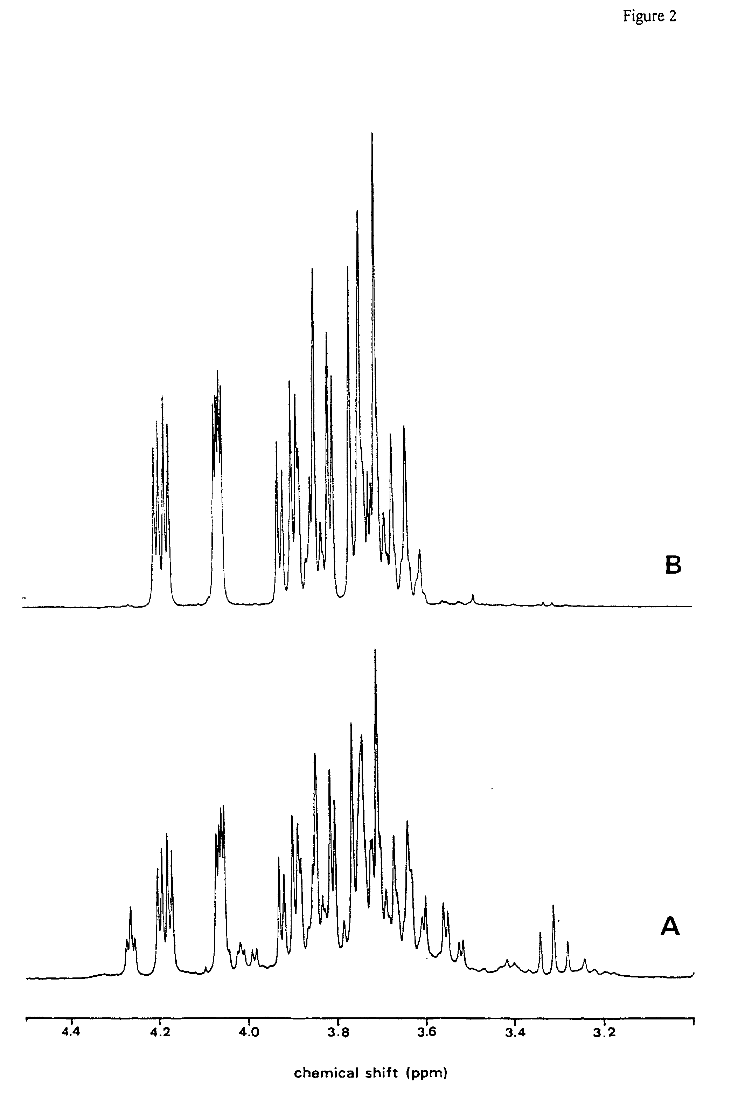

The degree of purification after each step was evaluated by ¹H-NMR spectroscopy. Figure 2 shows a comparison between the proton spectrum of an extract (A) and that of purified mannosylglycerate (B). The yield of 2-*O*-β―mannosylglycerate was approximately 2.5 g per 100 g of *Pyrococcus furiosus* cells paste (wet weight). The potassium concentration measured by plasma emission spectroscopy in the final solution of 2-*O*-β-mannosylglycerate (prepared to a final concentration of 1 M in the organic solute), was 1 M. No residual NH₄⁺ was detected in the solution of 2-*O*-β-mannosylglycerate utilized for evaluation of protection against stress factors. The 2-*O*-β-mannosylglycerate solution (potassium salt) was aliquoted and stored at -20°C until used. The thermal stability of mannosylglycerate was monitored by incubating the pure compound (pH 7.0, 300 mM) at 95°C in an oil bath and recording ¹H-NMR spectra at time intervals over a period of 180 min. No modifications in the spectrum were detected throughout the experiment, neither extra resonances were detected.

### EXAMPLE 2

This example shows that 2-*O*-β-mannosylglycerate has a thermoprotective effect on enzymes used as model-systems, namely rabbit muscle lactate dehydrogenase (LDH), yeast alcohol dehydrogenase (ADH), and bovine liver glutamate dehydrogenase (GDH) and can be used to protect enzymes in general against thermal deactivation.

Lactate dehydrogenase, LDH (Sigma Type III, rabbit muscle), purchased as a suspension in ammonium sulfate, was centrifuged, the supernatant was discarded, and the enzyme was suspended in 50 mM potassium phosphate pH 7.5. Alcohol dehydrogenase (Sigma, baker's yeast) and glutamate dehydrogenase (Sigma Type III, bovine liver) were obtained in the lyophilized form and used without further purification. Enzymes were assayed using a Olis spectrophotometer with a thermostated cell compartment at 30°C.

In the experiments where thermal stress was imposed, enzymes were prepared at a concentration of 50 µg . ml⁻¹ in 20 mM potassium phosphate buffer, and the reaction mixtures were placed in eppendorf tubes, incubated in a water-bath at the indicated temperatures, withdrawn at appropriate time intervals, cooled in an ice-bath and assayed immediately under standard conditions. All solutes were used at a final concentration of 500 mM.

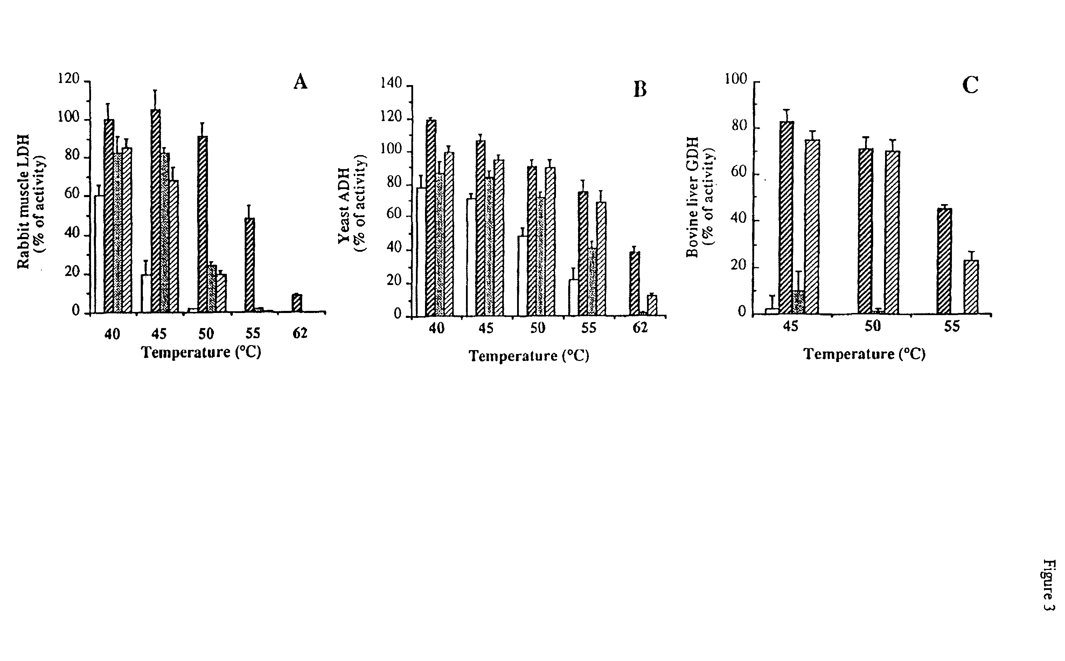

The effect of 2-*O*-β-mannosylglycerate on the thermostability of rabbit muscle LDH, baker's yeast ADH, and bovine liver GDH was compared to that of trehalose, a compound that is generally described as a thermostabilizer. Potassium chloride was always added as a control, since the potassium salt of 2-*O*-β-mannosylglycerate was used throughout the experiments. The percentage of residual activity in relation to pre-incubation activity, measured after 10 min of incubation at the temperatures indicated, is shown in Figure 3, for rabbit muscle LDH (A), yeast ADH (B), and bovine liver GDH (C). 2-*O*-β-Mannosylglycerate exerted a protective effect on lactate dehydrogenase at all temperatures, but its effectiveness was remarkable at the higher temperatures tested; in fact, after 10 min of incubation at 50°C, LDH retained only 5% of activity in the absence of 2-*O*-β-mannosylglycerate whereas in its presence 90% activity was recovered; the corresponding values found after incubation at 62°C were 0% and 10%, respectively. Furthermore, a comparison of the effect exerted by 2-*O*-β-mannosylglycerate with that of trehalose, showed that this solute was not as efficient as 2-*O*-β-mannosylglycerate as thermoprotector of LDH. Alcohol dehydrogenase was intrinsically more stable than lactate dehydrogenase; furthermore, the activity retained after 10 min incubation at temperatures ranging from 40 to 55°C was similar in the presence of 2-*O*-β-mannosylglycerate or KCl. However, after incubation at 62°C, the percentage of activity determined was significantly higher in the presence of the former compound: 40% and 10% activity was measured after incubation with 2-*O*-β―mannosylglycerate and KCl, respectively. Bovine liver GDH was extremely thermolabile; activity was almost completely lost after incubation for 10 min at 45°C; 2-*O*-β-mannosylglycerate was an effective protectant against thermal inactivation after incubation of the enzyme at supraoptimal temperatures; for example, incubation for 10 min at 55°C, resulted in a residual activity of 45% or 23% in the presence of 2-*O*-β-mannosylglycerate or KCl, respectively.

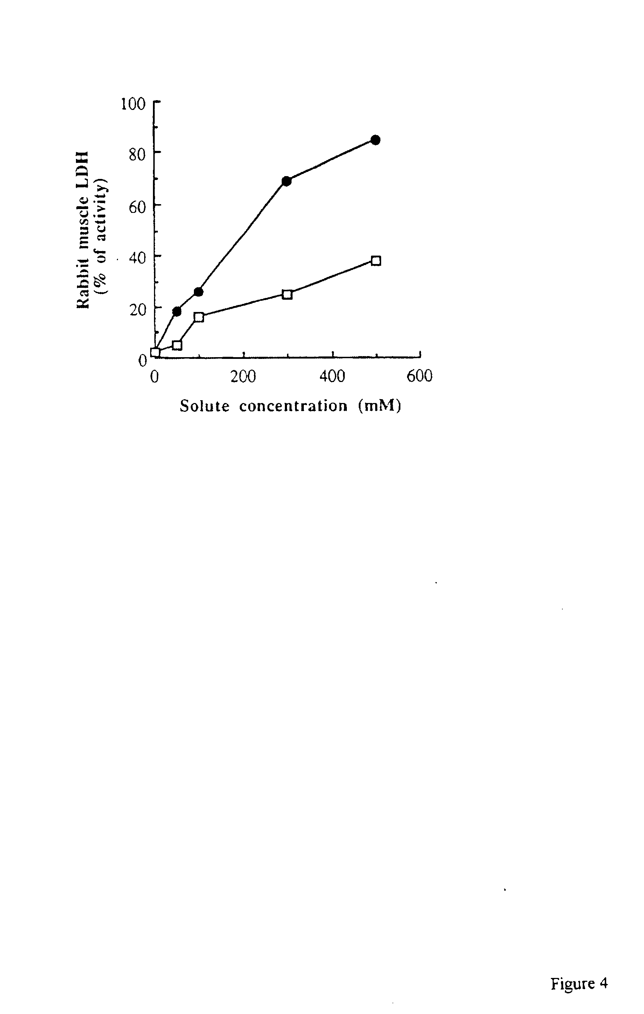

The effect of the concentration of 2-*O*-β-mannosylglycerate on the thermal stability of LDH was also monitored, and the results obtained after 10 min incubation at 50°C are shown in Figure 4. Increased concentrations of 2-*O*-β-mannosylglycerate were increasingly effective in protecting LDH against thermal stress. Concentrations of 2-*O*-β-mannosylglycerate higher than 500 mM were not tested; however, the increase in protection was much more pronounced when the concentration of 2-*O*-β-mannosylglycerate was increased from 100 to 300 mM, when compared to the effect when the concentration was further increased to 500 mM; this suggests "saturation" of the effect on LDH for a concentration of 2-*O*-β-mannosylglycerate around 500 mM. KCl was much less effective than 2-*O*-β-mannosylglycerate in protecting LDH against thermal stress; at temperatures above 50°C, no protection was provided by any of the concentrations of KCl tested.

### EXAMPLE 3

This example shows that 2-*O*-β-mannosylglycerate can be used as a protector against the deleterious effects of lyophilization and desiccation on the enzyme activity of the model enzymes rabbit muscle LDH and yeast ADH, and can be used for the protection of enzymes and enzymatic activity in general from the above mentioned effects.

The assay mixtures for freeze-drying experiments were prepared at an enzyme concentration of 50 µg . ml⁻¹, in 20 mM potassium phosphate buffer at the appropriate pH. The potassium salt of 2-*O*-β-mannosylglycerate, KCl or trehalose were added to obtain final concentrations of 500 mM. Enzyme activity was measured under standard conditions, the volume of each sample was determined, and all samples in a given experiment were frozen simultaneously by immersion in liquid nitrogen for 60 s. The frozen samples were lyophilized for 24 h on a lyophilizer and rehydrated in water to the same volume. The residual activity was immediately measured by standard methods, and the results were expressed as percentual activity with respect to the results obtained before freeze-drying.

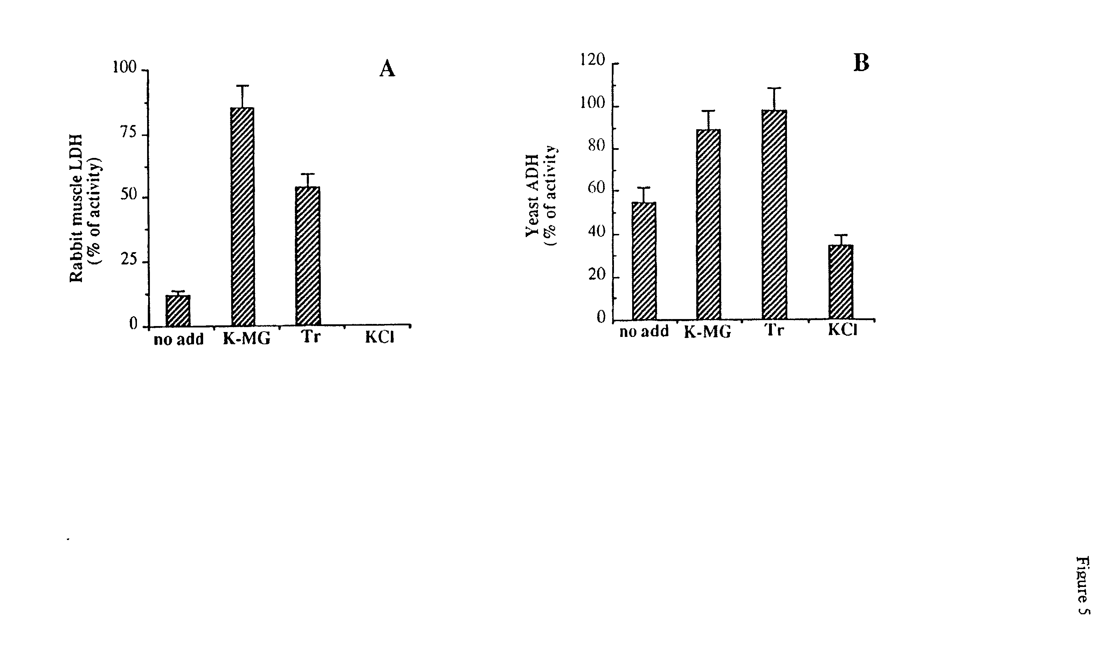

The effect of 2-*O*-β-mannosylglycerate on enzymic activity after freeze-drying of LDH (rabbit muscle) and ADH (yeast) are shown in Figure 5. LDH was very sensitive to freeze-drying (A); only 12% of the initial activity was retained after freeze-drying in the absence of added solutes. In contrast, when 2-*O*-β-mannosylglycerate or trehalose were added, the enzyme retained 85% and 54% of initial activity, respectively. Furthermore, the enzyme was completely inactivated after freeze-drying in the presence of KCl. Yeast ADH retained 55% activity in the absence of solutes (B), and this value was increased to 89% and 98% in the presence of 2-*O*-β-mannosylglycerate or trehalose, respectively.

## Claims

1. The use of 2-*O*-β-mannosylglycerate or 2-*O*-α-mannosylglycerate to protect cellular components or cells against stressing conditions, namely caused by heat, desiccation, freeze-drying, storage, transportation or repetitive use.

2. The use of 2-*O*-β-mannosylglycerate or 2-*O*-α-mannosylglycerate according to claim 1 to protect lactate dehydrogenase, alcohol dehydrogenase, and glutamate dehydrogenase, and any other enzyme or protein of microbial, plant or animal sources, against stress induced by heat, purification, transportation, or storage.

3. The use of 2-*O*-β-mannosylglycerate or 2-*O*-α-mannosylglycerate according to claim 1 to protect the activity of polymerase chain reaction (PCR) enzymes for clinical, biological and industrial purposes during storage, as well as during the high temperature recycling of the enzymes.

4. The use of 2-*O*-β-mannosylglycerate or 2-*O*-α-mannosylglycerate according to claim 1 to protect enzymes during lyophilization, desiccation and storage at low temperatures.

5. The use of 2-*O*-β-mannosylglycerate or 2-*O*-α-mannosylglycerate according to claim 1 to protect test kit enzymes for diagnostic, biological and industrial purposes during the manufacture, storage, and assays.

6. The use of 2-*O*-β-mannosylglycerate or 2-*O*-α-mannosylglycerate) according to claim 1 to protect enzymes or other proteins during their routine utilization for clinical, biological and industrial purposes, namely when used in biosensors.

7. The use of 2-*O*-β-mannosylglycerate or 2-*O*-α-mannosylglycerate according to claim 1 to protect cell membranes, liposomes, liposome-containing cosmetics or lipid related substances against stress such as desiccation and lyophilization.

8. The use of 2-*O*-β-mannosylglycerate or 2-*O*-α-mannosylglycerate according to claim 1 as an additive to cosmetics to protect skin against dehydration.

9. The use of 2-*O*-β-mannosylglycerate or 2-*O*-α-mannosylglycerate according to claim 1 to protect microbial cells and cellular tissues against damage caused by lyophilization, desiccation, high temperatures, and freezing.

## Patentansprüche

1. Verwendung von 2-O-β-Mannosylglycerat oder 2-O-α-Mannosylglycerat zum Schutz von Zellkomponenten oder Zellen gegen Belastungszustände, nämlich solche, die durch Wärme, Trocknen, Lyophilisierung, Lagerung Transport oder wiederholte Anwendung hervorgerufen werden.

2. Verwendung von 2-O-β-Mannosylglycerat oder 2-O-α-Mannosylglycerat nach Anspruch 1 zum Schutz von Lactatdehydrogenase, Alkoholdehydrogenase und Glutamindehydrogenase und irgendeinem anderen Enzym oder Protein mikrobiellen, pflanzlichen oder tierischen Ursprungs gegen Belastung, die durch Wärme, Reinigung, Transport oder Lagerung hervorgerufen wird.

3. Verwendung von 2-O-β-Mannosylglycerat oder 2-O-α-Mannosylglycerat nach Anspruch 1 zum Schutz der Aktivität von Enzymen zur Polymerase-Kettenreaktion (PCR) für klinische, biologische oder industrielle Zwecke während der Lagerung sowie während der Rückführung der Enzyme bei hohen Temperaturen.

4. Verwendung von 2-O-β-Mannosylglycerat oder 2-O-α-Mannosylglycerat nach Anspruch 1 zum Schutz von Enzymen während der Lyophilisierung, Trocknung und Lagerung bei niedrigen Temperaturen.

5. Verwendung von 2-O-β-Mannosylglycerat oder 2-O-α-Mannosylglycerat nach Anspruch 1 zum Schutz von Enzymen in Testkits für diagnostische, biologische und industrielle Zwecke während der Herstellung, Lagerung und der Untersuchung.

6. Verwendung von 2-O-β-Mannosylglycerat oder 2-O-α-Mannosylglycerat nach Anspruch 1 zum Schutz von Enzymen oder anderen Proteinen während ihrer Routine-Anwendung für klinische, biologische und industrielle Zwecke, nämlich bei Verwendung in Biosensoren.

7. Verwendung von 2-O-β-Mannosylglycerat oder 2-O-α-Mannosylglycerat nach Anspruch 1 zum Schutz von Zellmembranen, Liposomen, Liposome enthaltenden Kosmetika oder lipidähnlichen Substanzen gegen Belastung wie Trocknung und Lyophilisierung.

8. Verwendung von 2-O-β-Mannosylglycerat oder 2-O-α-Mannosylglycerat nach Anspruch 1 als Additiv für Kosmetika, um die Haut gegen eine Dehydratisierung zu schützen.

9. Verwendung von 2-O-β-Mannosylglycerat oder 2-O-α-Mannosylglycerat nach Anspruch 1 zum Schutz von mikrobiologischen Zellen und Zellgewebe gegen eine durch Lyophilisieren, Trocknen, hohe Temperaturen und Gefrieren verursachte Schädigung.

## Revendications

1. Utilisation de 2-O-β-mannosylglycérate ou de 2-O-α-mannosylglycérate pour protéger des composants cellulaires ou des cellules contre des conditions stressantes, à savoir des conditions provoquées par la chaleur, la dessiccation, la congélation, le stockage, le transport ou l'utilisation répétée.

2. Utilisation de 2-O-β-mannosylglycérate ou de 2-O-α-mannosylglycérate selon la revendication 1 pour protéger la lactate déshydrogénase, l'alcool déshydrogénase et la glutamate déshydrogénase, et toute autre enzyme ou protéine de sources microbiennes, végétales ou animales, contre le stress induit par la chaleur, la purification, le transport ou le stockage.

3. Utilisation de 2-O-β-mannosylglycérate ou de 2-O-α-mannosylglycérate selon la revendication 1 pour protéger l'activité d'enzymes de réaction d'amplification en chaîne par polymérase (ACP) à des fins cliniques, biologiques et industrielles pendant le stockage, ainsi que pendant le recyclage à haute température des enzymes.

4. Utilisation de 2-O-β-marnosylglycérate ou de 2-O-α-mannosylglycérate selon la revendication 1 pour protéger des enzymes pendant la lyophilisation, la dessiccation et le stockage à de basses températures.

5. Utilisation de 2-O-β-mannosylglycérate ou de 2-O-α-mannosylglycérate selon la revendication 1 pour protéger des enzymes de kit d'essai à des fins diagnostiques, biologiques et industrielles pendant la préparation, le stockage et les essais.

6. Utilisation de 2-O-β-mannosylglycérate ou de 2-O-α-mannosylglycérate selon la revendication 1 pour protéger des enzymes ou d'autres protéines pendant leur utilisation de routine à des fins cliniques, biologiques et industrielles, plus précisément lorsqu'elles sont utilisées dans des biocapteurs.

7. Utilisation de 2-O-β-mannosylglycérate ou de 2-O-α-mannosylglycérate selon la revendication 1 pour protéger des membranes cellulaires, des liposomes, des cosmétiques contenant des liposomes, ou des substances apparentées à des lipides contre un stress tel que la dessiccation et la lyophilisation.

8. Utilisation de 2-O-β-mannosylglycérate ou de 2-O-α-mannosylglycérate selon la revendication 1 comme additif à des cosmétiques pour protéger la peau contre la déshydratation.

9. Utilisation de 2-O-β-mannosylglycérate ou de 2-O-α-mannosylglycérate selon la revendication 1 pour protéger des cellules microbiennes et des tissus cellulaires contre des dommages provoqués par la lyophilisation, la dessiccation, les températures élevées et la congélation.
